# EUROPÄISCHE PATENTANMELDUNG

(11) **EP 4 624 973 A1**
(43) Veröffentlichungstag der Anmeldung: **01.10.2025**
(21) Anmeldenummer: 24167480.3
(22) Anmeldetag: 28.03.2024
(51) Int. Cl.: G01R 33/563, A61B 5/00

(54) **ELASTOGRAPHIE-VORRICHTUNG FÜR EINE MR-ELASTOGRAPHIE EINES KOPFES**

(71) Anmelder: Siemens Healthineers AG, 91301 Forchheim (DE); Centre National de la Recherche Scientifique (CNRS), 75016 Paris Cedex 16 (FR); Institut National de la Sante et de la Recherche Medicale (INSERM), 75013 Paris (FR); Université Paris XIII Paris-Nord, 93430 Villetaneuse (FR); Université Paris Cité, 75006 Paris (FR)
(72) Erfinder: SCHREGEL, Katharina, 69221 Dossenheim (DE); DARWISH, Omar, SE8 5EP London (GB); NEITZ, Gustav, 69221 Dossenheim (DE); ANNIO, Giacomo, 70042 Mola di Bari BA (IT); SINKUS, Ralph, 95620 Parmain (FR)
(74) Vertreter: Siemens Healthineers Patent Attorneys

(57) **Zusammenfassung**

Die Erfindung betrifft eine Elastographie-Vorrichtung ausgebildet zur Unterstützung einer Magnetresonanz-Elastographie eines Kopfes eines Untersuchungsobjektes (17). Die Elastographie-Vorrichtung umfasst ein erstes Auflageelement (11) ausgebildet zur Positionierung innerhalb einer Hochfrequenz-Empfangsspuleneinheit (19) und einen Schwingungserzeuger (21) ausgebildet zur Erzeugung mechanischer Wellen,
- wobei das erste Auflageelement (11) eine erste Aussparung (12) zur Aufnahme des Schwingungserzeugers (21) aufweist,
- das erste Auflageelement (11) eine zweite Aussparung (13) zur Aufnahme des Kopfes aufweist, und
- die erste Aussparung (12) derart geformt ist, dass diese den Schwingungserzeugers (21) zumindest teilweise passgenau umschließt und der Schwingungserzeuger (21) zumindest teilweise innerhalb der ersten Aussparung (12) angeordnet ist.

## Beschreibung

Die Erfindung betrifft eine Elastographie-Vorrichtung für ein Magnetresonanzgerät und eine Empfangseinheit mit einer Hochfrequenz-Empfangsspuleneinheit und mit einer Elastographie-Vorrichtung zur Verwendung in Kombination mit einem Magnetresonanzgerät, sowie ein Magnetresonanzgerät.

In einem Magnetresonanzgerät wird üblicherweise der zu untersuchende Körper eines Untersuchungsobjektes, insbesondere eines Patienten, mit Hilfe eines Hauptmagneten einem relativ hohen Hauptmagnetfeld, beispielsweise von 1,5 oder 3 Tesla, ausgesetzt. Im Rahmen einer Magnetresonanzbildgebung (MR-Bildgebung) werden mit Hilfe einer Gradientenspuleneinheit Gradientenpulse ausgespielt. Zusätzlich werden über eine Hochfrequenzantenneneinheit dann mittels geeigneter Antenneneinrichtungen hochfrequente Hochfrequenz-Pulse (HF-Pulse), insbesondere Anregungspulse, ausgesendet, was dazu führt, dass die Kernspins bestimmter, durch diese HF-Pulse resonant angeregter Atome um einen definierten Flipwinkel gegenüber den Magnetfeldlinien des Hauptmagnetfelds verkippt werden. Bei der Relaxation der Kernspins werden Hochfrequenz-Signale, so genannte Magnetresonanz-Signale, abgestrahlt, die mittels geeigneter Hochfrequenzantennen empfangen und dann weiterverarbeitet werden. Aus den so akquirierten Rohdaten können schließlich die gewünschten Bilddaten rekonstruiert werden. Für eine bestimmte Messung ist eine bestimmte Magnetresonanz-Steuerungssequenz (MR-Steuerungssequenz) auszusenden, welche aus einer Folge von Hochfrequenz-Pulsen, beispielsweise Anregungspulsen und Refokussierungspulsen, sowie passend dazu koordiniert auszusendenden Gradientenpulsen in verschiedenen Gradientenachsen entlang verschiedener Raumrichtungen besteht. Zeitlich passend hierzu werden Auslesefenster gesetzt, welche die Zeiträume vorgeben, in denen die induzierten Magnetresonanz-Signale erfasst werden.

Die Hochfrequenzantennen zum Empfang der Magnetresonanz-Signale sind typischerweise Teil einer Hochfrequenz-Empfangsspuleneinheit, welche möglichst nahe am zu untersuchenden Bereich des Untersuchungsobjektes angeordnet sind. Es gibt für verschiedene zu untersuchende Bereiche des Körpers dedizierte Hochfrequenz-Empfangsspuleneinheiten.

Bei der MR-Elastographie (MRE) wird die Tatsache ausgenutzt, dass die Phase der Magnetresonanz-Signale sich infolge von mechanischen Wellen ändert, die auf das Untersuchungsobjekt einwirken. Das Ausmaß dieser Änderung hängt von der Auslenkung (d. h. der Verschiebung aus der Ruhelage) des Gewebes infolge der mechanischen Wellen ab. Somit lässt sich aus den MR-Phasenbildern, d. h. Bildern, die die Phase der Kernmagnetisierung darstellen, Information über bestimmte mechanische Parameter des Gewebes ableiten, z. B. über die Elastizität. MRE ist demnach ein nicht-invasives Verfahren zur Quantifizierung der Elastizität und Steifigkeit eines Gewebes. Für die MRE ist zusätzlich zu einem herkömmlichen Magnetresonanzgerät ein Schwingungserzeuger zur Erzeugung der mechanischen Wellen, insbesondere im Untersuchungsbereich des Untersuchungsobjektes, erforderlich.

Insbesondere im Kopf können Veränderungen der Konsistenz des Gewebes neurologische Erkrankungen und/oder eine Pathologie indizieren. Zudem sind biomechanische Informationen von Hirntumoren für die Planung chirurgischer Eingriffe, zur Tumorcharakterisierung und Behandlungsüberwachung wertvoll. MRE im Bereich des Kopfes wird bislang lediglich im experimentellen Umfeld durchgeführt, insbesondere hinsichtlich Akquisitionsmethode, Vibrationsfrequenz und der verwendeten Geräte.

Der Erfindung liegt die Aufgabe zugrunde, eine besonders robuste und verlässliche Elastographie-Vorrichtung für MRE im Kopf anzugeben, welche zugleich bequem für den Patienten ist. Die Aufgabe wird durch die Merkmale der unabhängigen Ansprüche gelöst. Vorteilhafte Ausgestaltungen sind in den Unteransprüchen beschrieben.

Die erfindungsgemäße Elastographie-Vorrichtung, welche zur Unterstützung einer Magnetresonanz-Elastographie eines Kopfes eines Untersuchungsobjektes ausgebildet ist, umfasst ein erstes Auflageelement ausgebildet zur Positionierung innerhalb einer Hochfrequenz-Empfangsspuleneinheit und einen Schwingungserzeuger ausgebildet zur Erzeugung mechanischer Wellen, wobei
- das erste Auflageelement eine erste Aussparung zur Aufnahme des Schwingungserzeugers aufweist,
- das erste Auflageelement eine zweite Aussparung zur Aufnahme des Kopfes aufweist, und
- die erste Aussparung derart geformt ist, dass diese den Schwingungserzeugers zumindest teilweise passgenau umschließt und der Schwingungserzeuger zumindest teilweise innerhalb der ersten Aussparung angeordnet ist und/oder angeordnet werden kann.

Die erfindungsgemäße Elastographie-Vorrichtung ist vorzugsweise dazu ausgebildet, in Kombination mit einer Hochfrequenz-Empfangsspuleneinheit und einem Magnetresonanzgerät ein Verfahren zur MRE auszuführen. Die Hochfrequenz-Empfangsspuleneinheit ist typischerweise eine Kopfspuleneinheit, insbesondere eine Empfangsspuleneinheit ausgebildet zum Empfangen von MR-Signalen des Kopfes und/oder des Halses eines Untersuchungsobjekts bei Positionierung des Kopfes und/oder des Halses eines Untersuchungsobjekts zumindest teilweise innerhalb der Hochfrequenz-Empfangsspuleneinheit innerhalb eines Patientenaufnahmebereiches eines Magnetresonanzgerätes. Der Schwingungserzeuger ist dazu ausgebildet, mechanische Schwingungen, insbesondere mit Scherkomponenten und Kompressionskomponenten, auszugeben. Der Schwingungserzeuger kann beispielsweise wie in US7034534B2 beschrieben eine flexible Membran umfassen, welche mittels akustischer Energie zur Vibration angeregt wird. Der Schwingungserzeuger kann als Gravitations-Schwingungserzeuger ausgebildet sein.

Das erste Auflageelement kann als eine innerhalb einer Kopfspuleneinheit zu positionierende Auflagefläche und/oder Lagerungseinheit für einen Kopf eines Untersuchungsobjektes ausgebildet sein. Das erste Auflageelement kann beispielsweise Kunststoff und/oder Schaumstoff umfassen. Die erste Aussparung und die zweite Aussparung sind vorzugsweise an einer ersten Seite, insbesondere auf der Oberseite des ersten Auflageelementes angeordnet. Die entgegengesetzte Seite, insbesondere die Unterseite, kann entsprechend der Form der Hochfrequenz-Empfangsspuleneinheit ausgebildet sein. Ebenso kann die Form des ersten Auflageelementes entsprechend der Form der Hochfrequenz-Empfangsspuleneinheit ausgebildet sein, beispielsweise ovalförmig.

Die erste Aussparung ist typischerweise als Aushöhlung des ersten Auflageelementes ausgebildet. Die erste Aussparung ist vorzugsweise derart geformt, dass der Schwingungserzeuger zumindest teilweise passgenau von der ersten Aussparung umschlossen werden kann und/oder der Schwingungserzeuger bei Positionierung zumindest teilweise innerhalb der ersten Aussparung in stabiler Position gelagert und/oder gehalten wird. Die erste Aussparung kann demnach als Positionierungshilfe und/oder Lagerungshilfe für den Schwingungserzeuger dienen.

Die zweite Aussparung ist typischerweise als Aushöhlung des ersten Auflageelementes ausgebildet. Die zweite Aussparung ist vorzugsweise derart geformt, dass diese zumindest teilweise einen Kopf, insbesondere Hinterkopf, und/oder zumindest teilweise einen Hals des Untersuchungsobjektes aufnehmen kann. Die erste Aussparung und die zweite Aussparung sind typischerweise derart zueinander positioniert, dass bei Lagerung des Schwingungserzeugers in der ersten Aussparung und bei Positionierung eines Kopfes in der zweiten Aussparung der Schwingungserzeuger dazu ausgebildet ist, mechanische Wellen im Kopf zu initiieren. Die erste Aussparung und die zweite Aussparung weisen typischerweise einen Abstand von weniger als 6 cm, bevorzugt von weniger als 4 cm, besonders bevorzugt von weniger als 2 cm zueinander auf. Die erste Aussparung und die zweite Aussparung können disjunkt sein. Die erste Aussparung und die zweite Aussparung können ineinander übergehen und/oder miteinander verbunden sein. Bei Anordnung des Schwingungserzeugers und des Kopfes auf dem ersten Auflageelement weisen der Schwingungserzeuger und der Kopf typischerweise einen Abstand von weniger als 4 cm, bevorzugt einen Abstand von weniger als 3 cm, besonders bevorzugt einen Abstand von weniger als 2 cm, zueinander auf. Bei Anordnung des Schwingungserzeugers und des Kopfes auf dem ersten Auflageelement weisen der Schwingungserzeuger und der Kopf vorzugsweise einen Berührungspunkt und/oder eine Berührungsfläche zueinander auf. Bei Anordnung des Schwingungserzeugers und des Kopfes auf dem ersten Auflageelement kann zwischen dem Schwingungserzeuger und dem Kopf ein Kontaktelement, insbesondere ein Kissen, angeordnet sein.

Die erfindungsgemäße Elastographie-Vorrichtung ermöglicht eine präzise Positionierung des Schwingungserzeugers relativ zu dem zu positionierenden Kopf, sodass diese einen guten und zuverlässigen Kontakt zueinander aufweisen. Zudem ist der Schwingungserzeuger in der ersten Aussparung typischerweise selbständig stabil positionierbar, sodass dessen Position von einer Gewichtsbelastung durch den Kopf geschützt ist. Der Kopf ist zumindest teilweise innerhalb der zweiten Aussparung lagerbar, wobei dessen relative Position zum Schwingungserzeuger durch die zweite Aussparung vorgegeben werden kann. Dies ermöglicht eine besonders adäquate Penetration der vom Schwingungserzeuger erzeugten Wellen im Kopf. Insbesondere sind die Transversalwellen bei Ausbreitung frei von Wellen höherer Ordnung und weisen nur geringfügige Nichtlinearitäten auf. Die Elastographie-Vorrichtung ermöglicht demnach eine besonders robuste und verlässliche mechanische Anregung für MRE im Kopf, wobei zugleich insbesondere die zweite Aussparung eine bequeme Lagerung des Kopfes ermöglicht. Zudem kann die Elastographie-Vorrichtung in Kombination mit kommerziell verfügbaren Hochfrequenz-Empfangsspuleneinheiten verwendet werden, welche herkömmlich als Empfangsspuleneinheit für einen Kopf, auch ohne MRE, verwendbar sind. Diese Elastographie-Vorrichtung kann demnach als Zubehör verwendet werden und ist damit besonders kostengünstig und flexibel verwendbar.

Eine Ausführungsform der Elastographie-Vorrichtung sieht vor, dass die erste Aussparung derart ausgebildet ist, dass der Schwingungserzeuger reversibel löslich in der ersten Aussparung positionierbar ist. Der Schwingungserzeuger ist demnach flexibel und bedarfsgerecht in der ersten Aussparung anzuordnen. Insbesondere kann für die Positionierung des Kopfes auf dem ersten Auflageelement der Schwingungserzeuger entfernt und/oder zugleich positioniert werden. Zudem kann der Schwingungserzeuger gemäß dieser Ausführungsform für eine Reinigung und/oder Wartung der Elastographie-Vorrichtung entnommen werden. Dies ermöglicht eine besonders flexible Verwendung der Elastographie-Vorrichtung.

Eine Ausführungsform der Elastographie-Vorrichtung sieht vor, dass der Schwingungserzeuger als Gravitations-Schwingungserzeuger ausgebildet ist, welcher eine um eine Rotationsachse rotierbare exzentrische Masse und einen um eine zur Rotationsachse parallel gelagerte Antriebsachse drehbaren Schaft ausgebildet zum Antreiben der exzentrischen Masse aufweist, wobei die Antriebsachse und die Rotationsachse eine Antriebsebene bilden.

Der Gravitations-Schwingungserzeuger kann beispielsweise gemäß US20230305090A1 ausgebildet sein. US20230305090A1 offenbart auch die Funktionsweise eines derartigen Gravitations-Schwingungserzeugers. Ein derartiger Gravitations-Schwingungserzeuger ist besonders robust verwendbar und zeichnet sich insbesondere durch Stabilität im Bereich der Erzeugung mechanischer Wellen aus. Zudem ist ein derartiger Gravitations-Schwingungserzeuger besonders gut mit der Ansteuerung eines Magnetresonanzgeräts synchronisierbar.

Eine Ausführungsform der Elastographie-Vorrichtung sieht vor, dass das erste Auflageelement als Flächenelement in einer ersten Ebene ausgebildet ist, und die erste Aussparung derart ausgebildet ist, dass bei Positionierung des Schwingungserzeugers zumindest teilweise innerhalb der ersten Aussparung die Antriebsebene mit der ersten Ebene einen Winkel zwischen 25° und 65° einschließt.

Das Flächenelement kann eine Schaumstoffmatte umfassen, welche beispielsweise eine Höhe zwischen 0,5 cm und 7 cm, bevorzugt zwischen 1 cm und 5 cm, besonders bevorzugt zwischen 1,5 cm und 3 cm, aufweist. Die Höhe entspricht vorzugsweise der maximalen räumlichen Ausdehnung des ersten Auflageelementes senkrecht zur ersten Ebene. Die Oberseite des ersten Auflageelementes abzüglich der ersten Aussparung und der zweiten Aussparung, also unter Annahme einer aufgefüllten ersten Aussparung und zweiten Aussparung, kann der ersten Ebene entsprechen.

Die erste Ebene kann horizontal ausgerichtet sein. Die erste Aussparung und die zweite Aussparung sind vorzugsweise an einer ersten Seite, insbesondere auf der Oberseite des ersten Auflageelementes angeordnet. Die entgegengesetzte Seite des ersten Auflageelementes, insbesondere die Unterseite, kann entsprechend der Form der Hochfrequenz-Empfangsspuleneinheit geformt und/oder ausgebildet sein. Ebenso kann die Form des ersten Auflageelementes entsprechend der Form der Hochfrequenz-Empfangsspuleneinheit ausgebildet sein, beispielsweise ovalförmig.

Die Antriebsebene des Schwingungserzeugers schließt bei dessen Positionierung in der ersten Aussparung gemäß dieser Ausführungsform einen Winkel zwischen 25° und 65°, bevorzugt zwischen 35° und 55°, besonders bevorzugt zwischen 40° und 50°, mit der ersten Ebene ein. Die Antriebsebene des Schwingungserzeugers kann bei dessen Positionierung in der ersten Aussparung gemäß dieser Ausführungsform einen Winkel zwischen 43° und 47°, insbesondere auch von 45°, mit der ersten Ebene einschließen. Die erste Aussparung ermöglicht eine derartige Ausrichtung des Schwingungserzeugers auf Grundlage ihrer Form. Eine derartige Lagerung des Schwingungserzeugers ermöglicht ein Aussenden der mechanischen Wellen in drei Raumrichtungen und eine adäquate Penetration im Bereich des Kopfes.

Eine Ausführungsform der Elastographie-Vorrichtung sieht vor, dass die Antriebsachse einen geringeren Abstand zur ersten Ebene aufweist als die Rotationsachse. Die Rotationsachse und somit auch die exzentrische Masse weisen gemäß dieser Ausführungsform einen größeren Abstand zur ersten Ebene auf als die Antriebsachse. Dies ermöglicht eine Positionierung der exzentrische Masse besonders nahe an einem in der zweiten Aussparung gelagerten Kopf, was eine homogene Penetration des Kopfes mit mechanischen Wellen sicherstellt. Zudem ist der drehbare Schaft im Bereich der ersten Ebene angeordnet, insbesondere bei horizontaler Lagerung der Elastographie-Vorrichtung und/oder der ersten Ebene. Eine zur Ansteuerung des Schwingungserzeugers mit dem drehbaren Schaft verbundene flexible Drehzuleitung kann somit auf dem ersten Auflageelement und/oder teilweise in das erste Auflageelement integriert angeordnet werden und ergonomisch vom Untersuchungsbereich unter Berücksichtigung der Anatomie und/oder der Schultern weggeführt werden.

Eine Ausführungsform der Elastographie-Vorrichtung sieht vor, dass das erste Auflageelement als Flächenelement in einer ersten Ebene ausgebildet ist und der Schwingungserzeuger eine Gehäuseeinheit umfasst. Die Gehäuseeinheit schließt die vom Gravitations-Schwingungserzeuger umfassten Komponenten nach außen ab und weist zumindest eine planare Fläche auf. Bei Positionierung des Schwingungserzeugers zumindest teilweise innerhalb der ersten Aussparung schließt gemäß dieser Ausführungsform die planare Fläche mit der ersten Ebene einen Winkel zwischen 25° und 65°, bevorzugt zwischen 35° und 55°, besonders bevorzugt zwischen 40° und 50°, ein. Die planare Fläche kann mit der ersten Ebene einen Winkel zwischen 43° und 57°, insbesondere von 45°, einschließen. Die planare Fläche kann parallel zur Antriebsebene ausgebildet sein. Die planare Fläche kann als Ebene ausgebildet sein. Die erste Aussparung ist vorzugsweise derart ausgebildet, dass diese auf der der zweiten Aussparung zugewandten Seite für die planare Fläche zumindest teilweise passgenau geformt ist. Die Gehäuseeinheit kann quaderförmig, bevorzugt näherungsweise quaderförmig, ausgebildet sein. Die Gehäuseeinheit kann zwei planare Flächen aufweisen, wobei die vom Gravitations-Schwingungserzeuger umfassten Komponenten zwischen den zwei planaren Flächen angeordnet sind. Die Gehäuseeinheit weist typischerweise Seiten auf, die die zwei planaren Flächen abschließen. Die Gehäuseeinheit kann abgerundete Ecken und/oder abgerundete Kanten aufweisen. Die zwei planaren Flächen können parallel zueinander und/oder parallel zur Antriebsebene ausgestaltet sein. Diese Ausführungsform ermöglicht eine besonders anatomische Anordnung des Schwingungserzeugers an den Kopf unabhängig von der Form des Gehäuses des Schwingungserzeugers und eine homogene Penetration des Kopfes mit mechanischen Wellen.

Eine Ausführungsform der Elastographie-Vorrichtung sieht vor, dass das erste Auflageelement als Flächenelement in einer ersten Ebene ausgebildet ist, und die erste Aussparung derart ausgebildet ist, dass bei Positionierung des Schwingungserzeugers zumindest teilweise innerhalb der ersten Aussparung die Antriebsachse und/oder die Rotationsachse mit der ersten Ebene einen Winkel zwischen 2° und 20° bildet. Die Rotationsachse des Schwingungserzeugers schließt bei dessen Positionierung in der ersten Aussparung gemäß dieser Ausführungsform einen Winkel zwischen 2° und 20°, bevorzugt zwischen 3° und 10°, besonders bevorzugt zwischen 4° und 8°, mit der ersten Ebene ein. Die Rotationsachse des Schwingungserzeugers kann bei dessen Positionierung in der ersten Aussparung gemäß dieser Ausführungsform einen Winkel zwischen 4,5° und 6°, bevorzugt von 5° mit der ersten Ebene einschließen.

Diese Ausführungsform sieht demnach vor, dass der Schwingungserzeuger innerhalb der ersten Aussparung derart positionierbar ist, dass die Antriebsachse und die Rotationsachse nicht parallel zur ersten Ebene verlaufen. Insbesondere sieht diese Ausführungsform eine Verkippung der Rotationsachse relativ zur ersten Ebene vor. Dies ermöglicht aufgrund der Anatomie eines in der zweiten Aussparung positionierten Kopfes eine besonders gute Penetration der mechanischen Wellen im Kopf, insbesondere aufgrund einer potentiell besonders großen Berührungsfläche zwischen Kopf und Schwingungserzeuger.

Eine Ausführungsform der Elastographie-Vorrichtung sieht vor, dass die erste Aussparung derart ausgebildet ist, dass bei Positionierung des Schwingungserzeugers zumindest teilweise innerhalb der ersten Aussparung die Antriebsachse und/oder die Rotationsachse mit einer Geraden, welche zur craniocaudalen Achse eines zumindest teilweise innerhalb der zweiten Aussparung zu positionierenden Kopf des Untersuchungsobjektes parallel ausgebildet ist, einen Winkel zwischen 2° und 20° bildet.

Die craniocaudale Achse eines Kopfes verläuft typischerweise von der dem Hals zugewandten Seite des Kopfes zum oberen Kopfende, vorzugsweise mittig. Abhängig von der Positionierung des Kopfes innerhalb der zweiten Aussparung relativ zur ersten Ebene kann die craniocaudale Achse eines Kopfes parallel zur ersten Ebene oder verkippt zu dieser ausgerichtet sein. Gemäß dieser Ausführungsform ist die erste Aussparung demnach derart ausgebildet, dass der Schwingungserzeuger relativ zu dem Kopf des zu positionierenden Untersuchungsobjektes verkippt lagerbar ist. Die Rotationsachse schließt mit einer zur craniocaudalen Achse parallelen Geraden typischerweise einen Winkel zwischen 3° und 10°, bevorzugt zwischen 4° und 8°, besonders bevorzugt von 5° ein. Dies ermöglicht aufgrund der Anatomie eines in der zweiten Aussparung positionierten Kopfes eine besonders gute Penetration der mechanischen Wellen im Kopf, insbesondere aufgrund einer potentiell besonders großen Berührungsfläche zwischen Kopf und Schwingungserzeuger.

Eine Ausführungsform der Elastographie-Vorrichtung sieht vor, dass die Antriebsachse und/oder die Rotationsachse parallel zu einer linearen Verbindung zwischen einer caudal-anterioren Position und einer cranial-posterioren Position des zu positionierenden Kopfes ausgebildet ist. Insbesondere sind die erste Aussparung und die zweite Aussparung derart geformt, dass eine derartige relative Anordnung des Schwingungserzeugers zu einem Kopf möglich ist. Der Schwingungserzeuger ist gemäß dieser Ausführungsform derart gelagert, dass die Rotationsachse parallel zu einer Geraden verläuft, welche Gerade relativ zur craniocaudalen Achse in Richtung einer linearen Verbindung zwischen Kinn und Hinterkopf des Kopfes verkippt ist. Dies ermöglicht aufgrund der Anatomie eines in der zweiten Aussparung positionierten Kopfes eine besonders gute Penetration der mechanischen Wellen im Kopf, insbesondere aufgrund einer potentiell besonders großen Berührungsfläche zwischen Kopf und Schwingungserzeuger.

Eine Ausführungsform der Elastographie-Vorrichtung sieht vor, dass der Schwingungserzeuger eine flexible Drehzuleitung umfasst, welche flexible Drehzuleitung dazu ausgebildet ist, den drehbaren Schaft mit einem Schrittmotor zu verbinden und/oder eine Rotation von einem Schrittmotor an den drehbaren Schaft zu leiten. Die flexible Drehzuleitung ist typischerweise biegsam ausgebildet. Der Schrittmotor kann beispielsweise gemäß US20230296708A1 ausgebildet sein. Diese Ausführungsform ermöglicht eine flexible Ansteuerung des Schwingungserzeugers.

Eine Ausführungsform der Elastographie-Vorrichtung sieht vor, dass die flexible Drehzuleitung mit dem drehbaren Schaft eine Verbindung aufweist und die Verbindung einen Bajonettverschluss umfasst. Die Verbindung kann alternativ und/oder zusätzlich eine Sicherungsmutter umfassen. Ein Bajonettverschluss ist typischerweise reversibel löslich und ermöglicht demnach eine robuste und flexible Verbindung des Schwingungserzeugers mit dem Schrittmotor.

Eine Ausführungsform der Elastographie-Vorrichtung sieht vor, dass das erste Auflageelement als Flächenelement in einer ersten Ebene ausgebildet ist und das der flexiblen Drehzuleitung zugewandte Ende des drehbaren Schafts bei horizontaler Ansicht der ersten Ebene oberhalb des der flexiblen Drehzuleitung abgewandten Endes des drehbaren Schafts angeordnet ist. Bei horizontaler Positionierung des Untersuchungsobjektes derart, dass der Kopf innerhalb der zweiten Aussparung angeordnet ist, ist demnach gemäß dieser Ausführungsform das dem Kinn und/oder den Schultern zugewandte Ende der Rotationsachse und/oder der Antriebsachse oberhalb das dem Kopfende zugewandte Ende der Rotationsachse und/oder der Antriebsachse angeordnet. Typischerweise ist an dem Kinn und/oder den Schultern zugewandte Ende der Antriebsachse der drehbare Schaft für eine Verbindung zu der flexiblen Drehzuleitung angeordnet. Die flexible Drehzuleitung kann somit auf dem ersten Auflageelement und/oder teilweise in das erste Auflageelement integriert angeordnet werden und ist besonders ergonomisch zugänglich, auch wenn ein Kopf bereits in der zweiten Aussparung angeordnet ist. Diese Ausführungsform ermöglicht eine besonders einfache Verbindung zwischen flexibler Drehzuleitung und dem drehbaren Schaft.

Eine Ausführungsform der Elastographie-Vorrichtung sieht vor, dass die Elastographie-Vorrichtung zusätzlich ein zweites Auflageelement umfasst. Das zweite Auflageelement ist ausgebildet zur Positionierung auf einer Patientenlagerungsvorrichtung zur Unterstützung einer horizontalen Lagerung zumindest eines Oberkörpers des Untersuchungsobjektes, wobei das zweite Auflageelement eine dritte Aussparung aufweist, welche dritte Aussparung zur Aufnahme zumindest eines Teils der flexiblen Drehzuleitung ausgebildet ist.

Das zweite Auflageelement kann beispielsweise Kunststoff und/oder Schaumstoff umfassen. Die dritte Aussparung ist vorzugsweise an einer ersten Seite, insbesondere auf der Oberseite des zweiten Auflageelementes angeordnet. Die Oberseite des zweiten Auflageelementes ist dadurch gekennzeichnet, dass auf der Oberseite zumindest ein Oberkörper des Untersuchungsobjektes positionierbar ist. Die dritte Aussparung kann auch die volle Höhe des zweiten Auflageelementes durchdringen. Der Schrittmotor ist typischerweise außerhalb des Magnetresonanzgerätes und/oder des Patientenaufnahmebereiches und/oder der Detektoreinheit angeordnet. Die Hochfrequenz-Empfangsspuleneinheit, das erstes Auflageelement und der Schwingungserzeuger sind bei einer MRE-Untersuchung eines Untersuchungsobjektes typischerweise innerhalb des Patientenaufnahmebereiches angeordnet. Die flexible Drehzuleitung ermöglicht eine Transmission und/oder Weiterleitung der vom Schrittmotor erzeugten Impulse and den Schwingungserzeuger, also in den Patientenaufnahmebereich hinein. Die dritte Aussparung ermöglicht eine definierte Positionierung und einen definierten Verlauf der flexiblen Drehzuleitung. Die flexible Drehzuleitung kann vorzugsweise außerhalb des Betriebes der Elastographie-Vorrichtung flexibel in der dritten Aussparung positioniert werden. Die zumindest teilweise Lagerung der flexiblen Drehzuleitung in der dritten Aussparung ermöglicht eine bequeme Positionierung eines Untersuchungsobjektes auf dem zweiten Auflageelement und stellt einen stabilen Verlauf der flexiblen Drehzuleitung im Betrieb sicher, wobei Vibrationen reduziert und teils absorbiert werden können. Des Weiteren werden die flexible Drehzuleitung und der Schwingungserzeuger derart vom Untersuchungsobjekt abgekoppelt, dass eine Schädigung dieser Komponenten insbesondere aufgrund eines Gewichtes und/oder einer Gewichtsverlagerung des Untersuchungsobjektes vermieden werden können.

Eine Ausführungsform der Elastographie-Vorrichtung sieht vor, dass die Elastographie-Vorrichtung den Schrittmotor umfasst. Der Schrittmotor ist typischerweise mit einer Steuerungseinheit der Hochfrequenz-Empfangsspuleneinheit und/oder des Magnetresonanzgerätes verbunden. Dies ermöglicht eine Ansteuerung der Elastographie-Vorrichtung, insbesondere synchronisiert mit dem Magnetresonanzgerät.

Eine Ausführungsform der Elastographie-Vorrichtung sieht vor, dass die Elastographie-Vorrichtung zusätzlich ein Kissen umfasst, welches auf der ersten Ebene zwischen der ersten Aussparung und der zweiten Aussparung angeordnet ist. Insbesondere kann das Kissen zwischen dem Schwingungserzeuger und einem in der zweiten Aussparung zu positionierenden Kopf angeordnet sein und/oder angeordnet werden. Zwischen erster Aussparung und zweiter Aussparung kann eine weitere Aushöhlung des ersten Auflageelementes vorgesehen sein, in welcher weiteren Aushöhlung das Kissen positionierbar ist. Das Kissen kann ein Schaumstoffelement und/oder ein Federkissen umfassen. Das Kissen ist typischerweise als komprimierbares Kissen und/oder Gelkissen ausgebildet. Das Kissen umfasst typischerweise eine Hülle, welche Hülle ein Medium umschließt. Das Kissen, insbesondere die Hülle, ist vorzugsweise flexibel formbar. Das Kissen ist typischerweise flach ausgebildet und/oder weist eine Höhe von weniger als 3 cm, bevorzugt von weniger als 2 cm, besonders bevorzugt von weniger als 1 cm auf. Das Medium kann ein Gas, insbesondere Luft, oder ein Liquid, beispielsweise Wasser oder ein Gel, umfassen. Ein derartiges Kissen erhöht den Komfort des Untersuchungsobjektes, da ein direkter Kontakt des Schwingungserzeugers, welcher typischerweise rigide ausgebildet ist, und dem Kopf vermieden werden kann. Das Kissen ermöglicht zudem eine besonders gute Transmission der mechanischen Wellen, welche vom Schwingungserzeuger erzeugt werden können.

Des Weiteren geht die Erfindung aus von einer Empfangseinheit umfassend eine Hochfrequenz-Empfangsspuleneinheit und eine erfindungsgemäße Elastographie-Vorrichtung. Die Hochfrequenz-Empfangsspuleneinheit ist zum Empfangen von Magnetresonanz-Signalen eines Kopfes eines Untersuchungsobjektes ausgebildet und die Elastographie-Vorrichtung ist zumindest teilweise innerhalb der Hochfrequenz-Empfangsspuleneinheit angeordnet. Die Hochfrequenz-Empfangsspuleneinheit ist typischerweise als Kopfspule und/oder kombinierte Hals-/Kopfspule ausgebildet. Die Hochfrequenz-Empfangsspuleneinheit ist typischerweise dazu ausgebildet, den Kopf des Untersuchungsobjektes zumindest teilweise zu umschließen. Die Hochfrequenz-Empfangsspuleneinheit umfasst typischerweise zwischen 16 und 30 Empfangskanälen, vorzugsweise 20 Empfangskanäle. Insbesondere das erste Auflageelement ist zumindest teilweise innerhalb der Hochfrequenz-Empfangsspuleneinheit angeordnet. Das erste Auflageelement ist vorzugsweise frei von einer Verbindung zur Hochfrequenz-Empfangsspuleneinheit, insbesondere frei von einer permanenten Verbindung zu Hochfrequenz-Empfangsspuleneinheit. Das erste Auflageelement kann reversibel und/oder löslich innerhalb der Hochfrequenz-Empfangsspuleneinheit fixiert sein.

Des Weiteren geht die Erfindung aus von einem Magnetresonanzgerät umfassend eine Detektoreinheit und eine erfindungsgemäße Elastographie-Vorrichtung und/oder eine erfindungsgemäße Empfangseinheit. Die Detektoreinheit umfasst typischerweise einen Hauptmagneten, eine Hochfrequenzantenneneinheit und eine Gradientenspuleneinheit und ist zur Erzeugung von MR-Signales ausgebildet. Das erfindungsgemäße Magnetresonanzgerät ist zu einer MR-Elastographie des Kopfes ausgebildet. Das Magnetresonanzgerät ist vorzugsweise zu einer synchronisierten Ansteuerung der Elastographie-Vorrichtung und der Detektoreinheit ausgebildet.

Ausführungsformen der erfindungsgemäßen Empfangseinheit und des erfindungsgemäßen Magnetresonanzgerätes sind analog zu den Ausführungsformen der erfindungsgemäßen Elastographie-Vorrichtung ausgebildet. Die Vorteile der erfindungsgemäßen Empfangseinheit und des erfindungsgemäßen Magnetresonanzgerätes entsprechen im Wesentlichen den Vorteilen der erfindungsgemäßen Elastographie-Vorrichtung, welche vorab im Detail ausgeführt sind. Hierbei erwähnte Merkmale, Vorteile oder alternative Ausführungsformen können ebenso auch auf die anderen beanspruchten Gegenstände übertragen werden und umgekehrt.

Weitere Vorteile, Merkmale und Einzelheiten der Erfindung ergeben sich aus den im Folgenden beschriebenen Ausführungsbeispielen sowie anhand der Zeichnungen.

Es zeigen:
Fig. 1 eine Ausführungsform einer erfindungsgemäßen Elastographie-Vorrichtung in einer schematischen Darstellung,
Fig. 2 eine Ausführungsform eines Schwingungserzeugers in einer schematischen Darstellung,
Fig. 3 eine relative Position eines Schwingungserzeugers zum ersten Auflageelement in einer ersten Ansicht,
Fig. 4 eine relative Position eines Schwingungserzeugers zum ersten Auflageelement in einer zweiten Ansicht,
Fig. 5 einen auf dem ersten Auflageelement positionierten Kopf und Schwingungserzeuger in einer ersten Ansicht in einer schematischen Darstellung,
Fig. 6 einen auf dem ersten Auflageelement positionierten Kopf und Schwingungserzeuger in einer zweiten Ansicht in einer schematischen Darstellung, und
Fig. 7 eine Ausführungsform eines erfindungsgemäßen Magnetresonanzgerätes mit einer Ausführungsform einer erfindungsgemäßen Empfangseinheit in einer schematischen Darstellung.

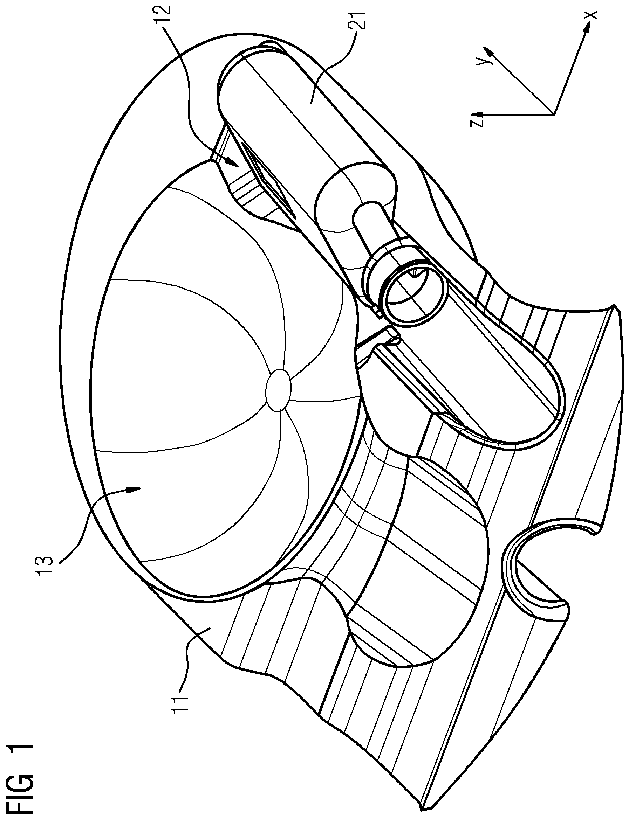

Figur 1 zeigt eine Ausführungsform einer erfindungsgemäßen Elastographie-Vorrichtung in einer schematischen Darstellung. Die dargestellte Elastographie-Vorrichtung ist zur Unterstützung einer Magnetresonanz-Elastographie eines Kopfes eines Untersuchungsobjektes 17 ausgebildet. Die Elastographie-Vorrichtung umfasst ein erstes Auflageelement 11, welches innerhalb einer nicht näher dargestellten Hochfrequenz-Empfangsspuleneinheit 19 positionierbar ist. Die Elastographie-Vorrichtung umfasst zudem einen Schwingungserzeuger 21, welcher zur Erzeugung mechanischer Wellen ausgebildet ist. Das erste Auflageelement 11 weist eine erste Aussparung 12 zur Aufnahme des Schwingungserzeugers 21 auf. Zudem weist das erste Auflageelement 11 eine zweite Aussparung 13 auf, welche derart geformt ist, dass darin ein Kopf zumindest teilweise positionierbar ist. Die erste Aussparung 12 ist derart geformt, dass diese den Schwingungserzeugers 21 zumindest teilweise passgenau umschließt. Der Schwingungserzeuger 21 ist zumindest teilweise innerhalb der ersten Aussparung 12 angeordnet. Dabei ist der Schwingungserzeuger 21 reversibel lösbar in der ersten Aussparung 12 positioniert und kann demnach bedarfsabhängig darin angeordnet und entnommen werden. Das erste Auflageelement 11 ist vorzugsweise als Flächenelement abzüglich der ersten Aussparung 12 und der zweiten Aussparung 13 ausgebildet. Die Position des Flächenelementes kann dabei durch eine erste Ebene angenähert werden, welche erste Ebene im dargestellten Fall der x-y-Ebene entspricht.

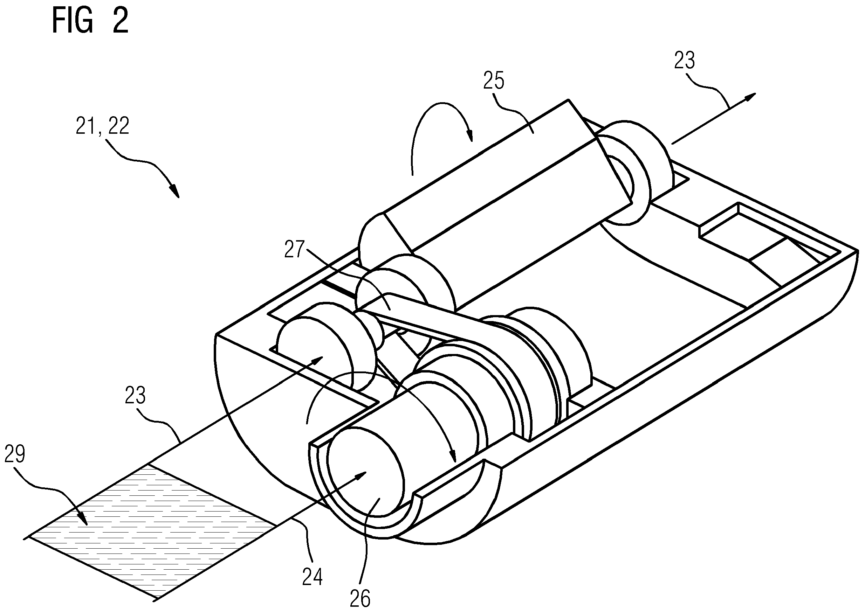

Figur 2 zeigt eine Ausführungsform eines Schwingungserzeugers 21 in einer schematischen Darstellung. Der Schwingungserzeuger 21 ist gemäß der dargestellten Ausführungsform als Gravitations-Schwingungserzeuger 22 ausgebildet. Der Gravitations-Schwingungserzeuger 22 umfasst eine um eine Rotationsachse 23 rotierbare exzentrische Masse 25. Zudem weist der Gravitations-Schwingungserzeuger 22 eine zur Rotationsachse 23 parallel gelagerte Antriebsachse 24 mit einem drehbaren Schaft 26 auf. Der drehbare Schaft 26 ist zum Antreiben der Rotationsachse 23 und damit zum Antreiben der exzentrischen Masse 25 ausgebildet. Hierfür ist die Antriebsachse 24 mit der Rotationsachse 23 über einen Riemen 27 verbunden. Die Antriebsachse 24 und die Rotationsachse bilden eine Antriebsebene 29.

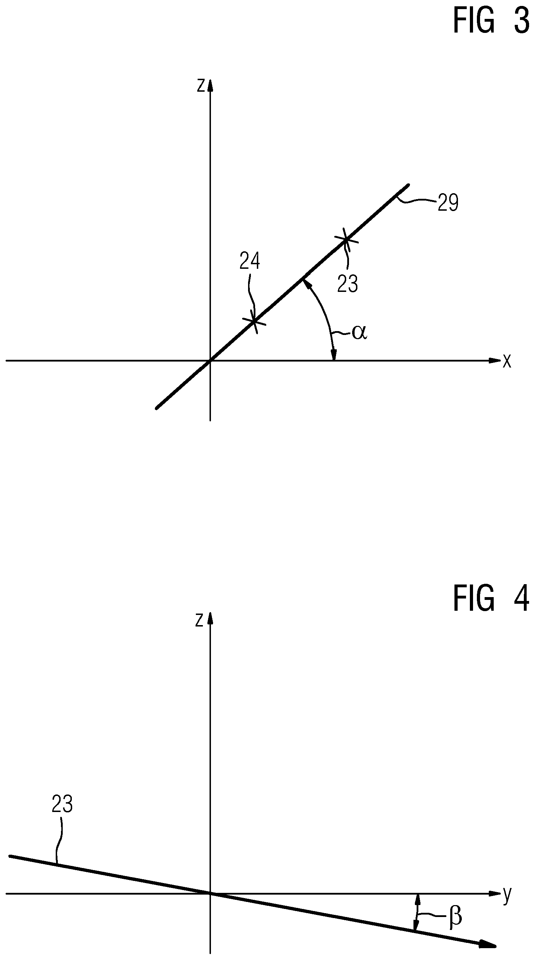

Figur 3 zeigt eine relative Position eines Schwingungserzeugers 21 zum ersten Auflageelement 11 in einer ersten Ansicht. Hierbei korrespondiert das Koordinatensystem mit dem in Figur 1 verwendeten Koordinatensystem. Der in Figur 2 dargestellte Schwingungserzeuger 21 ist gemäß dieser Ausführungsform zumindest teilweise innerhalb der ersten Aussparung 12 derart positioniert, dass die Antriebsebene 29 mit der ersten Ebene, also der x-y-Ebene, einen Winkel α zwischen 25° und 65°, im dargestellten Fall einen Winkel α von 45°, einschließt. Hierbei weist die Rotationsachse 23 einen größeren Abstand zur ersten Ebene, also der x-y-Ebene, auf, als die Antriebsachse 24.

Figur 4 zeigt eine relative Position eines Schwingungserzeugers 21 zum ersten Auflageelement 11 in einer zweiten Ansicht. Die zweite Ansicht ist senkrecht zur in Figur 3 verwendeten ersten Ansicht und das in Figur 4 verwendete Koordinatensystem korrespondiert mit dem in Figur 1 verwendeten Koordinatensystem. Der in Figur 2 dargestellte Schwingungserzeuger 21 ist gemäß dieser Ausführungsform zumindest teilweise innerhalb der ersten Aussparung 12 derart positioniert, dass die Rotationsachse 23, und damit auch die Antriebsachse 24, mit der ersten Ebene, also der x-y-Ebene, einen Winkel β zwischen 2° und 20°, im dargestellten Fall einen Winkel β von 10°, einschließt. Die craniocaudale Achse des Kopfes kann innerhalb der ersten Ebene, also der x-y-Ebene liegen. Die craniocaudale Achse des Kopfes kann mit der ersten Ebene, also der x-y-Ebene, genau einen Schnittpunkt aufweisen. Die Antriebsachse 24 und/oder die Rotationsachse 23 des Schwingungserzeugers 21, 22 können gemäß dieser Ausführungsform zu einer Geraden, welche mit der craniocaudalen Achse eines zumindest teilweise innerhalb der zweiten Aussparung zu positionierenden Kopf des Untersuchungsobjektes 17 parallel ausgebildet ist, einen Winkel zwischen 2° und 20° bilden. Im dargestellten Fall sind zudem die Antriebsachse 24 und die Rotationsachse 25 parallel zu einer linearen Verbindung zwischen einer caudal-anterioren Position und einer cranial-posterioren Position des zu positionierenden Kopfes ausgebildet. Die Antriebsachse 24 und die Rotationsachse 25 sind demnach relativ zur ersten Ebene höchstens 20° in Richtung des Kinnes des Kopfes verkippt.

Die erste Aussparung 12 ist vorzugsweise derart ausgestaltet, dass das dem Kinn des zu positionierenden Kopfes zugewandte Ende des Schwingungserzeugers 21 auf der gleichen Seite der ersten Ebene, also der x-y-Ebene, wie auch das Kinn angeordnet ist und/oder in Richtung der z-Achse das gleiche Vorzeichen aufweist wie das Kinn und/oder die Nase des Kopfes. Die erste Aussparung 12 ist vorzugsweise derart ausgestaltet, dass das dem cranialen Ende des Kopfes zugewandten Ende des Schwingungserzeugers 21 auf der entgegengesetzten Seite der ersten Ebene, also der x-y-Ebene, wie auch das Kinn angeordnet ist und/oder in Richtung der z-Achse das entgegengesetzte Vorzeichen aufweist wie das Kinn und/oder die Nase des Kopfes.

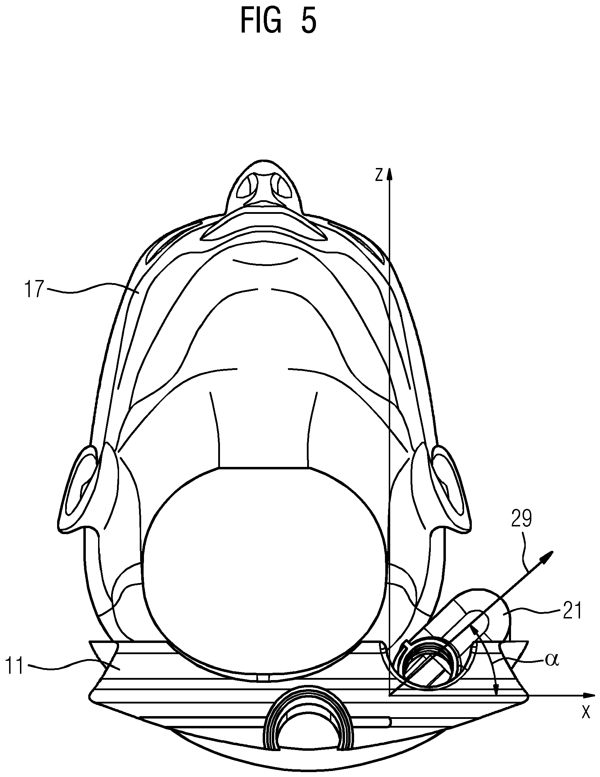

Figur 5 zeigt einen auf dem ersten Auflageelement 11 positionierten Kopf des Untersuchungsobjektes 17 und Schwingungserzeuger 21 in einer ersten Ansicht in einer schematischen Darstellung analog zu Figur 3.

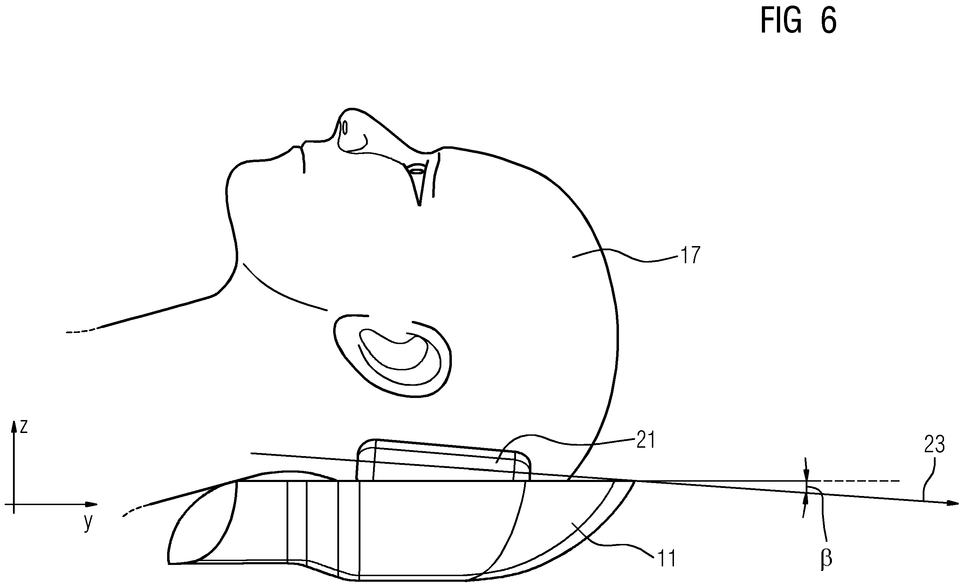

Figur 6 zeigt einen auf dem ersten Auflageelement 11 positionierten Kopf des Untersuchungsobjektes 17 und Schwingungserzeuger 21 in einer zweiten Ansicht in einer schematischen Darstellung analog zu Figur 4.

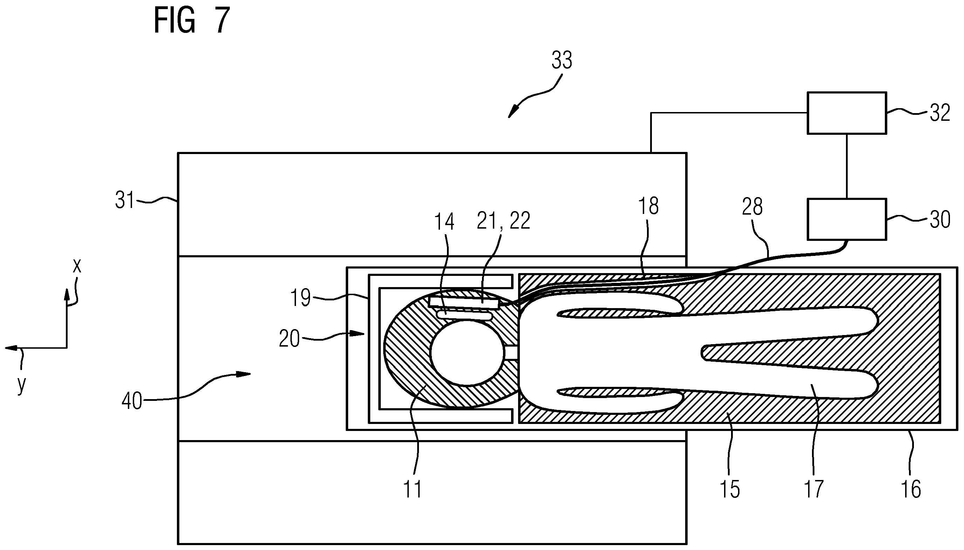

Figur 7 zeigt eine Ausführungsform eines erfindungsgemäßen Magnetresonanzgerätes 33 mit einer Ausführungsform einer erfindungsgemäßen Empfangseinheit 20 in einer schematischen Darstellung. Das erfindungsgemäße Magnetresonanzgerät 33 umfasst gemäß dieser Ausführungsform eine hohlzylinderförmige Detektoreinheit 31 umschließend, insbesondere konzentrisch umgebend, einen zylinderförmigen Patientenaufnahmebereich 40. Der zylinderförmige Patientenaufnahmebereich 40 ist zu einer Aufnahme eines Untersuchungsobjektes 17 ausgebildet. Die Längsachse des zylinderförmigen Patientenaufnahmebereiches 40 entspricht der y-Achse gemäß Figur 1. Das Untersuchungsobjekt 17 kann mittels einer Patientenlagerungsvorrichtung 16 des Magnetresonanzgeräts 33 in den Patientenaufnahmebereich 40 geschoben werden. Die Detektoreinheit 31 umfasst typischerweise einen nicht näher dargestellten Hauptmagneten, eine nicht näher dargestellte Gradientenspuleneinheit und/oder eine nicht näher dargestellte Hochfrequenzantenneneinheit ausgebildet zum Aussenden von Anregungspulsen.

Zu einer Steuerung der Detektoreinheit 31 weist das Magnetresonanzgerät 33 eine Steuerungseinheit 32 auf. Die Steuerungseinheit 32 steuert zentral das Magnetresonanzgerät 33, wie beispielsweise das Durchführen von MR-Steuerungssequenzen. Zudem umfasst die Steuerungseinheit 32 eine nicht näher dargestellte Rekonstruktionseinheit zu einer Rekonstruktion von medizinischen Bilddaten. Die Steuerungseinheit 32 kann eine nicht näher dargestellte Anzeigeeinheit und eine nicht näher dargestellte Eingabeeinheit aufweisen. Zudem kann die Steuerungseinheit 32 zur Auswertung von MRE-Daten ausgebildet sein.

Die Empfangseinheit 20 umfasst eine Hochfrequenz-Empfangsspuleneinheit 19, welche zum Empfangen von Magnetresonanz-Signalen eines Kopfes eines Untersuchungsobjektes 17 ausgebildet ist. Die Hochfrequenz-Empfangsspuleneinheit 19 ist in der dargestellten Ausführungsform als Kopfspule ausgebildet, welche dazu ausgebildet ist, den Kopf und/oder den Nacken eines Untersuchungsobjektes 17 zumindest teilweise käfigartig zu umgeben. Die Hochfrequenz-Empfangsspuleneinheit 19 weist vorzugsweise mehrere Empfangsspulen und/oder Empfangskanäle auf.

Zudem umfasst die Empfangseinheit 20 eine erfindungsgemäße Elastographie-Vorrichtung, die zumindest teilweise innerhalb der Hochfrequenz-Empfangsspuleneinheit 19 angeordnet. Die in Figur 5 dargestellte Ausführungsform der Elastographie-Vorrichtung umfasst einen als Gravitations-Schwingungserzeuger 22 ausgebildeten Schwingungserzeuger 21 mit einer flexiblen Drehzuleitung 28. Die flexible Drehzuleitung 28 ist dazu ausgebildet, den drehbaren Schaft 26 mit einem Schrittmotor 30 zu verbinden und eine Rotation von einem Schrittmotor 30 an den drehbaren Schaft 26 zu leiten. Hierzu weist die flexible Drehzuleitung 28 mit dem drehbaren Schaft 26 eine Verbindung umfassend einen nicht näher dargestellten Bajonettverschluss auf. Der Schrittmotor 30 wird gemäß dieser Ausführungsform von der Elastographie-Vorrichtung umfasst. Zudem weist der Schrittmotor 30 mit der Steuerungseinheit 32 eine Verbindung auf. Die Steuerungseinheit 32 kann zur Ansteuerung der Elastographie-Vorrichtung ausgebildet sein. Die Steuerungseinheit 32 ist vorzugsweise zur Ansteuerung des Schrittmotors 30 und/oder zur Synchronisation des Schrittmotors 30 mit einer von der Detektoreinheit 31 auszugebenden MR-Steuerungssequenz ausgebildet. Zudem kann die Steuerungseinheit 32 zur Auswertung der von der Hochfrequenz-Empfangsspuleneinheit 19 empfangenen Magnetresonanz-Signale unter Berücksichtigung von Informationen hinsichtlich der Ansteuerung der Elastographie-Vorrichtung ausgebildet sein.

Gemäß dieser Ausführungsform umfasst die Elastographie-Vorrichtung zudem ein zweites Auflageelement 15 ausgebildet zur Positionierung auf der Patientenlagerungsvorrichtung 16 des Magnetresonanzgerätes 33. Die Patientenlagerungsvorrichtung 16 ist dabei zur Unterstützung einer horizontalen Lagerung zumindest eines Oberkörpers des Untersuchungsobjektes 17 ausgebildet und das zweite Auflageelement 15 kann zwischen der Patientenlagerungsvorrichtung 16 und einem horizontal liegenden Untersuchungsobjekt 17 positioniert werden. Das zweite Auflageelement 15 weist eine dritte Aussparung 18 auf, welche dritte Aussparung 18 zur Aufnahme zumindest eines Teils der flexiblen Drehzuleitung 28 ausgebildet ist. Zudem umfasst die Elastographie-Vorrichtung gemäß dieser Ausführungsform ein Kissen 14, welches zwischen der ersten Aussparung 12 und der zweiten Aussparung 13, insbesondere zwischen dem Schwingungserzeuger 21 und einem zu positionierenden Kopf, angeordnet werden kann.

Das dargestellte Magnetresonanzgerät 33 kann selbstverständlich weitere Komponenten umfassen, die Magnetresonanzgeräte 33 gewöhnlich aufweisen. Eine allgemeine Funktionsweise eines Magnetresonanzgeräts 33 ist zudem dem Fachmann bekannt, so dass auf eine detaillierte Beschreibung der weiteren Komponenten verzichtet wird.

Obwohl die Erfindung im Detail durch die bevorzugten Ausführungsbeispiele näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen. Unabhängig vom grammatikalischen Geschlecht eines bestimmten Begriffes sind Personen mit männlicher, weiblicher oder anderer Geschlechteridentität mit umfasst.

## Patentansprüche

1. Elastographie-Vorrichtung ausgebildet zur Unterstützung einer Magnetresonanz-Elastographie eines Kopfes eines Untersuchungsobjektes umfassend ein erstes Auflageelement ausgebildet zur Positionierung innerhalb einer Hochfrequenz-Empfangsspuleneinheit und einen Schwingungserzeuger ausgebildet zur Erzeugung mechanischer Wellen, wobei
- das erste Auflageelement eine erste Aussparung zur Aufnahme des Schwingungserzeugers aufweist,
- das erste Auflageelement eine zweite Aussparung zur Aufnahme des Kopfes aufweist, und
- die erste Aussparung derart geformt ist, dass diese den Schwingungserzeugers zumindest teilweise passgenau umschließt und der Schwingungserzeuger zumindest teilweise innerhalb der ersten Aussparung angeordnet ist.

2. Elastographie-Vorrichtung nach Anspruch 1,
wobei die erste Aussparung derart ausgebildet ist, dass der Schwingungserzeuger reversibel löslich in der ersten Aussparung positionierbar ist.

3. Elastographie-Vorrichtung nach einem der vorangehenden Ansprüche,
wobei der Schwingungserzeuger als Gravitations-Schwingungserzeuger ausgebildet ist, welcher eine um eine Rotationsachse rotierbare exzentrische Masse und einen um eine zur Rotationsachse parallel gelagerte Antriebsachse drehbaren Schaft ausgebildet zum Antreiben der exzentrischen Masse aufweist, wobei die Antriebsachse und die Rotationsachse eine Antriebsebene bilden.

4. Elastographie-Vorrichtung nach Anspruch 3,
wobei das erste Auflageelement als Flächenelement in einer ersten Ebene ausgebildet ist, und die erste Aussparung derart ausgebildet ist, dass bei Positionierung des Schwingungserzeugers zumindest teilweise innerhalb der ersten Aussparung die Antriebsebene mit der ersten Ebene einen Winkel zwischen 25° und 65° einschließt.

5. Elastographie-Vorrichtung nach Anspruch 4,
wobei die Antriebsachse einen geringeren Abstand zur ersten Ebene aufweist als die Rotationsachse.

6. Elastographie-Vorrichtung nach einem der Ansprüche 3 bis 5,
wobei das erste Auflageelement als Flächenelement in einer ersten Ebene ausgebildet ist und der Schwingungserzeuger eine Gehäuseeinheit umfasst, welche Gehäuseeinheit die vom Gravitations-Schwingungserzeuger umfassten Komponenten nach außen abschließt, und welche Gehäuseeinheit zumindest eine planare Fläche aufweist, wobei bei Positionierung des Schwingungserzeugers zumindest teilweise innerhalb der ersten Aussparung die planare Fläche mit der ersten Ebene einen Winkel zwischen 25° und 65° einschließt.

7. Elastographie-Vorrichtung nach einem der Ansprüche 3 bis 6,
wobei das erste Auflageelement als Flächenelement in einer ersten Ebene ausgebildet ist, und die erste Aussparung derart ausgebildet ist, dass bei Positionierung des Schwingungserzeugers zumindest teilweise innerhalb der ersten Aussparung die Antriebsachse und/oder die Rotationsachse mit der ersten Ebene einen Winkel zwischen 2° und 20° bildet.

8. Elastographie-Vorrichtung nach einem der Ansprüche 3 bis 7,
wobei der Schwingungserzeuger eine flexible Drehzuleitung umfasst, welche flexible Drehzuleitung dazu ausgebildet ist, den drehbaren Schaft mit einem Schrittmotor zu verbinden und/oder eine Rotation von einem Schrittmotor an den drehbaren Schaft zu leiten.

9. Elastographie-Vorrichtung nach Anspruch 8,
wobei das erste Auflageelement als Flächenelement in einer ersten Ebene ausgebildet ist und das der flexiblen Drehzuleitung zugewandte Ende des drehbaren Schafts bei horizontaler Ansicht der ersten Ebene oberhalb des der flexiblen Drehzuleitung abgewandten Endes des drehbaren Schafts angeordnet ist.

10. Elastographie-Vorrichtung nach einem der Ansprüche 8 bis 9,
wobei die flexible Drehzuleitung mit dem drehbaren Schaft eine Verbindung aufweist und die Verbindung einen Bajonettverschluss umfasst.

11. Elastographie-Vorrichtung nach einem der Ansprüche 8 bis 10,
zusätzlich umfassend den Schrittmotor.

12. Elastographie-Vorrichtung nach einem der Ansprüche 8 bis 11,
zusätzlich umfassend ein zweites Auflageelement ausgebildet zur Positionierung auf einer Patientenlagerungsvorrichtung zur Unterstützung einer horizontalen Lagerung zumindest eines Oberkörpers des Untersuchungsobjektes, wobei das zweite Auflageelement eine dritte Aussparung aufweist, welche dritte Aussparung zur Aufnahme zumindest eines Teils der flexiblen Drehzuleitung ausgebildet ist.

13. Elastographie-Vorrichtung nach einem der vorangehenden Ansprüche,
zusätzlich umfassend ein Kissen angeordnet auf der ersten Ebene zwischen der ersten Aussparung und der zweiten Aussparung.

14. Empfangseinheit umfassend eine Hochfrequenz-Empfangsspuleneinheit ausgebildet zum Empfangen von Magnetresonanz-Signalen eines Kopfes eines Untersuchungsobjektes und eine Elastographie-Vorrichtung nach einem der vorangehenden Ansprüche, wobei die Elastographie-Vorrichtung zumindest teilweise innerhalb der Hochfrequenz-Empfangsspuleneinheit angeordnet ist.

15. Magnetresonanzgerät umfassend eine Detektoreinheit und eine Elastographie-Vorrichtung nach einem der Ansprüche 1 bis 13 und/oder eine Empfangseinheit nach Anspruch 14.
